# EUROPEAN PATENT SPECIFICATION

(11) **EP 3 687 481 B1**
(45) Date of publication and mention of the grant of the patent: **28.12.2022**
(21) Application number: 18756453.9
(22) Date of filing: 27.08.2018
(51) Int. Cl.: A61K 8/27, A61K 8/49, A61Q 5/00, A61K 8/64, A61K 8/65, A61K 8/66, A61Q 17/00, A61Q 19/10

(54) **AN ANTIMICROBIAL COMPOSITION**
ANTIMIKROBIELLE ZUSAMMENSETZUNG
COMPOSITION ANTIMICROBIENNE

(30) Priority: 29.09.2017 EP 17194025
(43) Date of publication of application: 05.08.2020
(73) Proprietor: Unilever Global IP Limited, Wirral, Merseyside CH62 4ZD (GB); Unilever IP Holdings B.V., 3013 AL Rotterdam (NL)
(72) Inventor: DAS, Somnath, Bangalore 560066 (IN); PRAMANIK, Amitava, Bangalore 560066 (IN)
(74) Representative: Tansley, Sally Elizabeth
(86) International application number: PCT/EP2018/072995
(87) International publication number: WO 2019/063222

(56) References cited:
- EP-A1- 2 281 816
- US-A1- 2004 191 331
- Johannes Baier ET AL: "Bio-inspired mineralization of zinc oxide in presence of ZnO-binding peptides", , 1 January 2012 (2012-01-01), pages 380-391, XP055314053, Retrieved from the Internet: URL:http://biointerfaceresearch.com/?downl oad=293 [retrieved on 2016-10-26]
- DANHONG YAN ET AL: "Characterization and Bacterial Response of Zinc Oxide Particles Prepared by a Biomineralization Process", JOURNAL OF PHYSICAL CHEMISTRY PART B: CONDENSED MATTER, MATERIALS, SURFACES, INTERFACES & BIOPHYSICAL, vol. 113, no. 17, 30 April 2009 (2009-04-30), pages 6047-6053, XP055313347, US ISSN: 1520-6106, DOI: 10.1021/jp808805w
- YIN PENG ET AL: "Polymer-Controlled Crystallization of Zinc Oxide Hexagonal Nanorings and Disks", JOURNAL OF PHYSICAL CHEMISTRY PART B: CONDENSED MATTER, MATERIALS, SURFACES, INTERFACES & BIOPHYSICAL, vol. 110, no. 7, 1 February 2006 (2006-02-01), pages 2988-2993, XP055313670, US ISSN: 1520-6106, DOI: 10.1021/jp056246d
- OSAMU YAMAMOTO: "Influence of particle size on the antibacterial activity of zinc oxide", INTERNATIONAL JOURNAL OF INORGANIC MATERIALS, vol. 3, no. 7, 1 November 2001 (2001-11-01), pages 643-646, XP055043689, ISSN: 1466-6049, DOI: 10.1016/S1466-6049(01)00197-0
- Satyawati S Joshi: "Crystal Habit Modification Using Habit Modifiers", , 2 July 2012 (2012-07-02), XP055439182, Retrieved from the Internet: URL:http://cdn.intechopen.com/pdfs/26216.p df [retrieved on 2018-01-09]

## Description

### Field of the invention

This invention relates to an antimicrobial composition. The invention more particularly relates to a personal care or cleansing composition e.g those for care or cleansing of hair or body which provides anti-microbial efficacy. It more particularly relates to a cleansing composition for hair and scalp comprising actives that interact to provide synergistic antimicrobial efficacy for anti-dandruff benefits.

### Background of the invention

The invention relates to an anti-microbial composition useful for cleansing of any body part but especially suitable for hair and scalp. Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts. To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g zinc pyrithione (ZPTO), octopirox (piroctone olamine), azole antimicrobials (e.g. climbazole), selenium sulfide and combinations thereof.

While the problem of dandruff is mitigated to a large extent through use of the above actives in such compositions, there is a need for enhancing the efficacy of these actives. The present inventors have found that the efficacy of one of the above actives, zinc pyrithione(ZPTO) can be enhanced when combined with habit modified crystals of zinc oxide preferably with those comprising entrapped particles of water soluble protein. The inventors further found that conventional zinc oxide which is not habit modified as per the present invention do not provide the synergy along with ZPTO.

Zinc oxide is a potent bacteriostatic agent, i.e., it can arrest the growth of bacteria on a substrate or in a medium, but it does not have appreciable bactericidal activity.

Crystals of zinc oxide exist in the form of hexagonal rods of different sizes. Recent publications indicate that 3D structures of zinc oxide make up the largest group, which includes shapes like nanorods, needles, helixes, springs, rings, ribbons, tubes, belts, wires and combs, dandelion, flower snowflakes, coniferous and urchin-like. Zinc oxide is also obtainable in 2D structures, such as nanoplate/nanosheet and nanopellets. Examples of one-dimensional structures include nanorods and nanowires.

Zinc oxide has been the subject of several patents and research articles covering various forms, morphologies, crystal structures and applications thereof.

Zinc oxide (ZnO) generally exists as hexagonal rods in the Wurtzite crystal structure where O²⁻ and Zn²⁺ ions are tetrahedrally coordinated and stacked alternately along the Z-axis. Polarity of each crystal face of ZnO is different. In a hexagonal crystal structure, the two hexagonal faces are Zn⁺² rich, consequently they are positively charged, while the six rectangular faces are less positively charged due to the presence of Zn⁺² and O⁻² ions. This also means that the hexagonal faces are polar as compared to the rectangular faces.

Attempts were made in the past to restrict/ modulate the growth of specific faces of ZnO crystal by use of certain molecules, which when present in the reaction medium selectively bind to specific crystal faces resulting in changes in either the shape or the size of the crystal or both.

CN104017393A [Anhui Henghao Science & Technology Co. Ltd, 2014] discloses a sericite/nano-zinc oxide composite material and a preparation method thereof. The surface of sericite powder is coated with nanoparticles of zinc oxide. The preparation method comprises adding calcium hydroxide or calcium oxide into a mixed system of the sericite powder and zinc sulfate solution to obtain the nano-zinc oxide coated sericite powder composite material. The material has good antibacterial performance.

US2004191331 (Procter and Gamble) discloses a composition comprising a particulate zinc material wherein the particulate zinc material has a crystallite size less than 600 A and a shampoo composition comprising an effective amount of a surfactant, an effective amount of a particulate zinc material, an effective amount of a metal salt of pyrithione, and an effective amount of a suspending agent wherein the particulate zinc material has a crystallite size less than 600 A.

US2004058855 (Procter and Gamble) discloses a method for delivering excess zinc to eukaryotic cells to inhibit the metabolism of the cell, the method comprising treating the cells with a zinc ionophoric that is capable of delivering a zinc ion across a cellular membrane wherein the minimum inhibitory concentration (MIC) of the zinc ionophoric material is less than about 500 ppm. Further disclosed is a method for delivering excess zinc to eukaryotic cells to inhibit the metabolism of the cell, the method comprising treating the cells with a zinc ionophoric material that is capable of delivering a zinc ion across a cellular membrane wherein the zinc ionophoric material is in combination with a zinc containing material and further wherein there is an increase in an intracellular zinc level by 1.5 fold more than would occur in the absence of the zinc ionophoric material.

Thus, to the knowledge of the present inventors, an antimicrobial composition comprising zinc pyrithione in combination with habit modified crystals of zinc oxide preferably comprising water soluble protein has not been known or published so far.

There is still need for newer and more efficacious compositions that synergistically enhance the antimicrobial efficacy of zinc pyrithione.

This is achieved by first preparing habit modified crystals of zinc oxide through use of water soluble proteins as crystal habit modifier via in-situ synthesis of zinc oxide particles. We have determined that proteins such as sericin (derived from silk), lysozyme and bovine serum albumin (BSA) help generate a novel morphology of crystals, which provide biocidal activity. We have determined that it is possible to modify the morphology, particle stacking and defects in the crystals by selective use of certain proteins as crystal habit modifiers. These proteins can bind to the crystal faces of ZnO and thereby influence the functionality of the crystals. In addition, we have determined that the proteins affect the crystal formation in the nucleation step resulting in an increase in defects in the crystals. When such a crystal modified zinc oxide is combined with zinc pyrithione synergistic antimicrobial activity especially against fungus like *Malassezia furfur* is observed.

### Summary of the invention

In accordance with the first aspect is disclosed an antimicrobial composition comprising
(a) habit modified crystals of zinc oxide; and
(b) zinc pyrithione
wherein the ratio of photoluminescence peak intensity at 452 nm of the habit modified crystals of zinc oxide to that of zinc oxide is at least 5.

The habit modified crystals of zinc oxide preferably comprise entrapped particles of a water soluble protein.

In accordance with a second aspect is disclosed a process to prepare a composition of the invention comprising the step of mixing zinc pyrithione with the habit modified crystals of zinc oxide wherein the habit modified crystals of zinc oxide are prepared by a process comprising the steps of:
(i) mixing aqueous solution of a water soluble protein with aqueous solution of a non-hydrolysable water-soluble zinc salt;
(ii) adding a weak base thereto;
(iii) heating the reaction mixture of step (ii) to attain temperature of 60 to 90 °C; and,
(iv) filtering the contents of step (iii), washing the residue with water and drying it to obtain said habit modified crystals of zinc oxide as the filtrate.

Other aspects of the invention are discussed in details in the description.

### Detailed description of the invention

According to a first aspect is disclosed an antimicrobial composition comprising habit modified crystals of zinc oxide and (b) zinc pyrithione; wherein the ratio of photoluminescence peak intensity at 452 nm of the habit modified crystals of zinc oxide to that of zinc oxide is at least 5.

ZnO typically crystallizes as hexagonal rod-like crystals, having eight faces of which two are regular hexagon and six are rectangular. Habit modification is a process in which additives are introduced in the reaction medium during the formation of zinc oxide crystals which can alter the growth of the crystals thereby allowing and inhibiting growth in particular directions. This results in anisotropy and hence the morphology changes. The term "habit modification" is used commonly in crystal engineering. As per the present invention the habit modified zinc oxide preferably comprises entrapped particles of one or more proteins which is water-soluble.

By water soluble, as used herein, is meant that the solubility of the material in water at 25°C and atmospheric pressure is 0.1% by weight or more.

It is particularly preferred that the protein is one or more of sericin, bovine serum albumin (BSA), gelatin, egg-albumin, casein, zein, whey or lysozyme, more preferably sericin, bovine serum albumin or lysozyme.

The morphology of zinc oxide crystals, the particle stacking and defects therein are modified by the selective use of the above proteins as crystal habit modifiers. Without wishing to be bound by theory, it is believed that these proteins, in particular the water-soluble proteins, can selectively bind to the crystal faces of the zinc oxide crystal, thereby influencing the functionality of the crystals. Further, it is believed that the proteins also affect the crystal formation in the nucleation step, which results in an increase in the defects in the crystals.

Crystalline materials normally crystallize from their solutions as having a shape that resembles their unit cell, the smallest unit of the material. Zinc oxide has the hexagonal unit cell and it typically crystallizes as hexagonal prismatic rods, with two hexagonal faces and six rectangular faces. Deliberate deviation of such natural morphology by playing with the crystallization is called crystal habit modification. It could be done by changing physical conditions like subjecting the system to different temperature, pressure, concentration or solvation conditions. Alternatively, one may employ traces of impurities that are known to preferentially adsorb on specific faces of the crystals thereby preventing growth on those faces. These impurities are called crystal habit modifiers. They often remain adsorbed on the faces of the crystals.

In the present invention, proteins, in particular water-soluble ones are used as crystal habit modifiers that are believed to be responsible for deviation in the growth of zinc oxide from its natural hexagonal prismatic morphology to agglomerated fine thin disk-shaped particles forming spherical assemblies.

The habit modified crystals of zinc oxide of the invention are capable of industrial application *inter-alia* in the broad technical fields of home and personal care products. Non-limiting examples of specific fields include skin cleansing, hair care, surface hygiene and household care products.

Zinc oxide is used in several cosmetic compositions but the usual zinc oxide and at least some of its modified variants do not possess sufficient antimicrobial activity. Some of such zinc oxides have been used and disclosed in US2004191331 viz. USP-2, USP-1, Z-Cote, and Nanox. The present inventors have experimented with similar types of zinc oxides (having large surface areas) and determined that they do not exhibit the synergistic antimicrobial activity with ZPTO that are achieved through use of the composition disclosed and claimed in the present invention. Without wishing to be bound by theory, the present inventors believe that the synergistic benefit is achieved through use of zinc oxides having specific structural defects achieved through the use of proteins in the present invention and that the large surface area of the zinc oxides used in US2004191331 are not the critical factors in achieving the desired benefits when used along with ZPTO.

It is a general practice to add known antimicrobial agents to top-up the antibacterial activity. The present invention provides a synergistic mixture of zinc pyrithione and the habit modified crystals of zinc oxide, which has significant antimicrobial efficacy even at low levels.

The habit modified crystals of zinc oxide comprising entrapped particles of protein are preferably shaped like a disc. The thickness of each disc is preferably 10 nm to 30 nm. The average diameter of the discs is preferably in the range of 200 nm to 500 nm. The average size is measured by Malvern Multisizer^{®}, but any equivalent device may also be used. The zinc oxide particles are preferably in the form of spherical assemblies of average size 0.8 µm to 3 µm.

A habit modified crystals of zinc oxide preferably has a formula ZnOₓ where x<1 and characterized by a photoluminescence peak at 452 nm which is several times higher (more than a factor of 5) than that obtained from conventional zinc oxides. Preferably x has a value in the range of 0.95 to 1. The composition of the invention preferably comprises 0.05 to 5%, more preferably 0.10 to 2.0%, further more preferably 0.2 to 1.0%, most preferably 0.3 to 0.5%by weight of the habit modified crystals of zinc oxide.

The composition of the invention comprises zinc pyrithione (ZPTO) which is shorthand for zinc 1-hydroxy-2-pyridinethione. The polyvalent metal salt of pyrithione is represented by the following general formula:

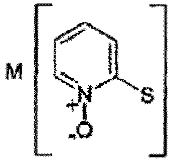

In the case of zinc pyrithione, M is the metal cation zinc.

Zinc pyrithione is preferably present in 0.01 to 10%, more preferably 0.01 to 5.0%, further more preferably from 0.05 to 2.0% based on weight of the composition. ZPTO is a particulate material. While the particle size is not critical to achieve the benefits of the present invention, the particle size of ZPTO is preferably from 0.25 to 8 micrometer, more preferably from 0.5 to 8.0 micrometer, and further more preferably from 1.0 to 7.5 micrometer. ZPTO is commercially available from Kolon Life Science Inc., Sino Lion (USA) Ltd, Lonza, and other suppliers.

The composition of the invention preferably additionally comprises a cosmetically acceptable carrier. The carrier is preferably chosen such that the composition of the invention can be delivered for use as a shampoo, a hair conditioner or a body wash. As per one aspect the cosmetically acceptable carrier is water or an aqueous solution. According to another preferred aspect, the carrier additionally comprises a surfactant. The cosmetically acceptable vehicle is such that the composition can be prepared as a shampoo, conditioner, body wash, hand wash or face wash product, cream, lotion, gel, powder, ointment, or a soap bar

According to a further preferred aspect of the present invention, the composition is either a shampoo, a hair conditioner, or a body wash product.

As per an especially preferred aspect of the invention, the composition is a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of the composition is preferably equal to or higher than 4.0, more preferably in the range of 5.0 to 10.0.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives.

Suspending agent, if included, will generally be present in a shampoo composition of the invention at levels of from 0.1 to 10%, preferably from 0.5 to 6%, more preferably from 0.5 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The composition of the invention is preferably aqueous based. It preferably comprises high amounts of water preferably from 70 to 95% by weight of the composition.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

The composition of the invention may be used for skin care e.g. body, hand or face wash. The antimicrobial composition may further comprise a surfactant. The preferred surfactants are nonionic surfactants, such as C₈-C₂₂, preferably C₈-C₁₆ fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Thus, in a highly preferred aspect, the antimicrobial compositions include the surfactant selected from the group of anionic surfactant, fatty acid amide, alkyl sulphate, linear alkyl benzene sulphonate, and combinations thereof.

When the surfactants are present, the antimicrobial composition preferably comprises 1 to 90% surfactant by weight of the composition

When surfactant is used, a particularly preferred surfactant is soap. Soap is a suitable surfactant for personal washing applications of the antimicrobial composition of the invention. The soap is preferably C₈-C₂₄ soap, more preferably C₁₀-C₂₀ soap and most preferably C₁₂-C₁₈ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also be used in other desired proportions.

When present, the soap, of the present is preferably present in an amount of 1 to 90%, preferably from 10 to 85%, more preferably 25 to 75% by weight of the composition. Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, furthermore preferably at least 5% by weight of the composition. When water is the carrier, a preferred liquid composition comprises 10 to 99.8% by weight water.

A preferred process to prepare the composition of the invention comprises the step of mixing zinc pyrithione with the habit modified crystals of zinc oxide prepared by a process comprising the steps of:
(i) mixing aqueous solution of a water soluble protein with aqueous solution of a non-hydrolysable water-soluble zinc salt;
(ii) adding a weak base thereto;
(iii) heating the reaction mixture of step (ii) to attain temperature of 60 to 90 °C; and,
(iv) filtering the contents of step (iii), washing the residue with water and drying it to obtain said habit modified crystals of zinc oxide as the filtrate.

It is preferred that the non-hydrolysable water-soluble zinc salt is a nitrate, sulphate, acetate or formate. The salt is preferably not a chloride salt. Use of chloride salt with the protein leads to undesirable complexation between zinc ions and the amino acids contained within the proteins.

The term "hydrolysable salt" means salts, which instantly (within five minutes of addition to water) forms either zinc oxide or basic oxy- hydroxides of zinc when added to water. Examples of hydrolysable salts of zinc include anhydrous zinc chloride, zinc complexes of substituted amino alcohols and sodium zincate. It is believed that hydrolysable salts form ZnO particles in the medium therefore, it is not possible to modulate the morphology/ defects/crystallinity when such salts are used.

It is preferred that the weak base is hexamethylenetetramine, urea or magnesium hydroxide. Other preferred weak bases include ammonia, pyridine, hydroxylamine and methylamine. It is necessary that water-soluble protein is made to contact with the non-hydrolysable water soluble zinc salt, before the weak base is introduced into the reaction vessel. This ensures proper complexation of the zinc ions with the amino acid residues in the protein. It is also preferred that the reaction mix, prior to introduction of the weak base, is heated to 40 °C to 60 °C for about 30 minutes. On the other hand, if the base is contacted with the zinc salt prior to addition of the protein, it results in the formation of very small seed crystals of hexagonal zinc oxide particles in the medium. The resultant product may have significantly lower photoluminescence and photo-catalytic activity.

Strong bases, such as sodium hydroxide, should not be used in the process because there is likelihood of faster precipitation.

Upon addition of the weak base, the reaction mixture of step (ii) is heated to attain a temperature of 60 to 90 °C. In a preferred aspect of the method, the heated reaction mixture of step (iii) is autoclaved under pressure of 4 to 6 bar in an inert atmosphere. Autoclaving leads to a more efficacious product as compared to its non-autoclaved counterpart.

In the final step, the contents of step (iii) is filtered by any suitable means. The residue is washed with water and thereafter it is dried to obtain the crystal habit modified zinc oxide as the filtrate.

It is further preferred that the crystal habit modified zinc oxide is calcined at 600 °C to 800 °C. Calcination is also used to imply a thermal treatment process in the absence or limited supply of air or oxygen applied to ores and other solid materials to bring about a thermal decomposition, phase transition, or removal of a volatile fraction. Calcination normally takes place at temperatures below the melting point of a given material.

It is preferred that the photoluminescence emission (at 452 nm wavelength) of the calcined product is at least 8 for a 0.5 mg/mL solution of the product.

Also disclosed in accordance with this invention is non-therapeutic use of the composition for delivering anti-microbial benefits.

Also disclosed is a non-therapeutic use of habit modified crystals of zinc oxide; and zinc pyrithione;
wherein the ratio of photoluminescence peak intensity at 452 nm of the habit modified crystals of zinc oxide to that of zinc oxide is at least 5; and
wherein the habit modified crystals of zinc oxide are shaped like a disc; the thickness of each disc is from 10 nm to 30 nm and the average diameter of the disc is in the range of 200 nm to 500 nm for manufacture of a composition for providing antimicrobial benefit.

The following non-limiting examples further illustrate preferred embodiments of the invention. All percentages referred to in the examples and throughout this specification are based on total weight unless otherwise indicated.

### Examples

Example A: One gram commercially available sericin was added to 50 ml distilled water. The contents were stirred well until a clear solution was obtained. To this solution, an aqueous solution of 3 g zinc nitrate hexahydrate was added. The solution was heated at 40 °C for 15 minutes. 1.5 g of hexamine was dissolved in 50 ml of water. Hexamine solution was added to the mixture. The contents were transferred to a stoppard hydrothermal bottle and heated in an air oven at 90 °C for 5 hours. under pressure of 5.0 bar in an atmosphere of nitrogen. The habit modified crystals of zinc oxide that were formed, were filtered, washed with water and air-dried.

Example B: A part of the product of Example A was calcined at 700 °C for two hours to prepare sample of Example B.

Example C: The sample was zinc pyrithione sourced from sigma-aldrich.

Example D: ZnO commercially available from Merck as EMSURE^{®} ACS,Reag brand + ZPTO at a weight ratio of 1:2.

Calcination of commercially available ZnO was done at 700 °C for two hours.

Example 1: Example A + Example C at a weight ratio of 1:2

Example 2: Example B + Example C at weight ratio of 1:2

The above samples were subjected to their anti fungal efficacy on *M. furfur* as described hereinafter.

### Antifungal activity:

M. furfur ATCC 14521 strain was revived from glycerol stock on Modified Dixon agar. Plate was at 30°C for 72 hrs. Approximately 72 hrs prior to testing, the plate culture was inoculated onto the surface of another sterile Modified Dixon agar plate & incubated at 30°C for approximately 72 hrs. From second subculture, the optical density of culture is adjusted at 620 nm to give cell strength of 10⁷ cfu/ml. The culture was further diluted for 100 times to achieve 10⁵ cfu/ml in Pityrosporum broth.

10 mg of each sample (the sample is particulate hence it has to be thoroughly mixed such as vortex/ sonication) was weighed into a sterile 1.5 ml Eppendorf tube. 1000 µL of M. furfur inocula were added to the above tubes and a blank sterile 1.5 ml Eppendorf tube (no particles as control), respectively so that the load in each tube is 10⁵ cells/ml.

All the samples were kept shaken @120 RPM in a shaker incubator at 32°C. After 24 hours incubation, 10 µL of the samples were withdrawn from the respective 1.5 ml Eppendorf tubes and diluted with 990 µL of Pityrosporum broth. This represents 10⁻² dilution. Serial dilution was performed from the above 24 hours inocula and plated 100 µL of dilution onto modified Dixon plate. Plate dilution 10⁻², 10⁻³, 10⁻⁴ and 10⁻⁵. All the plates were kept for incubation at 32°C for 24 - 72 hrs. Plate count for all the plates were done.

The data on the antifungal activity in terms of log reduction is summarized in Table - 1 below:

**Table - 1:**

| Example | Sample | Contact time, hr | Log reduction |
|---|---|---|---|
| A | Uncalcined sample of habit modified crystals of ZnO | 24 | 1.07 |
| B | Calcined sample of habit modified crystals of ZnO | 24 | 1.07 |
| C | ZPTO | 24 | 1.58 |
| D | Calcined commercial ZnO + ZPTO(weight ratio 1:2) | 24 | 1.87 |
| 1 | Example A + Example C (weight ratio 1:2) | 24 | 2.27 |
| 2 | Example B + Example C (weight ratio 1:2) | 24 | 2.91 |

The data in Table - 1 indicates that a composition as per the invention (Example 1 and 2) provide vastly superior efficacy as compared to those ourside the invention (Examples A to D).

### Examples B, C and 2 to 4: Anti fungal efficacy of compositions at various weight ratios of habit modified crystals of ZnO and ZPTO:

Various compositions as shown in Table -2 below (at various weight ratios) were tested for anti-fungal efficacy using the same procedure as above. The data is summarized in Table -2. The data for Examples B, C and 2 have been repeated for comparison purposes.

**Table - 2:**

| Example | Sample | Contact time, hr | Log reduction |
|---|---|---|---|
| B | Calcined sample of habit modified crystals of ZnO | 24 | 1.07 |
| C | ZPTO | 24 | 1.58 |
| 2 | Example B + Example C (weight ratio 1:2) | 24 | 2.91 |
| 3 | Example B + Example C (weight ratio 1:1) | 24 | 2.11 |
| 4 | Example B + Example C (weight ratio 2:1) | 24 | 1.77 |

The data in the table above indicates that there is improved efficacy over a wide range of weight ratios of ZnO of the invention and ZPTO but the weight ratio is preferably less than 1:1.

### Anti bacterial efficacy of the composition of the invention:

Compositions as per Table -3 were prepared and they were tested for antibacterial efficacy as per the procedure given below:

An aqueous stock solution (1% w/v) of the composition was prepared. 8ml of the solution was taken. The pH maintained at 10. This was followed by taking 1 ml cell (1 mL bacterial suspension of Staphylococcus aureus (stock 10⁹ cells/mL). In this 1 ml of DI water was added further. The suspensions were incubated for 30 and 60 seconds at room temperature. To 1ml of the above reaction mixture 9ml of Dey-Engley Neutralizing Broth was added and incubated for 5 mins. This represents 10⁻¹ dilution. Serial dilutions were done and plated 1ml onto LB plate.

**Table 3**

| Example | Sample | Contact time, seconds | Log reduction |
|---|---|---|---|
| C | ZPTO | 30 | 2.6 |
| 2 | Example B + Example C (weight ratio 1:2) | 30 | 3.3 |

The data in table-3 clearly demonstrates the antibacterial activity of the composition of the invention.

Photoluminescence spectra of various samples of calcined zinc oxide at 452 nm at 0.5 mg/ml concentration was measured and the data in intensity (a.u) is summarized in the table-4 below:

**Table 4**

| Example | Sample | Photoluminescence intensity (a.u) at 452 nm | Ratio |
|---|---|---|---|
| F | Calcined sample of commercial ZnO (as used in Example D) | 3 | - |
| B | Calcined sample of crystals of ZnO habit modified with Sericin (as in Example A) | 24 | 8 |
| 4 | Calcined sample of crystals of ZnO habit modified with bovine serum albumin | 17 | 5.7 |

The data in the table 4 above indicates that the habit modified zinc oxide prepared as per the present invention (using proteins and further calcined) exhibits a ratio of photoluminescence as compared to calcined samples of conventional zinc oxide of more than 5.

## Claims

1. An antimicrobial composition comprising
(a) habit modified crystals of zinc oxide; and
(b) zinc pyrithione
wherein the ratio of photoluminescence peak intensity at 452 nm of the habit modified crystals of zinc oxide to that of zinc oxide is at least 5; and
wherein the habit modified crystals of zinc oxide are shaped like a disc; the thickness of each disc is from 10 nm to 30 nm and the average diameter of the disc is in the range of 200 nm to 500 nm.

2. An antimicrobial composition as claimed in claim 1 comprising
(a) Habit modified crystals of zinc oxide comprising entrapped particles of a water soluble protein; and
(b) Zinc pyrithione.

3. A composition as claimed in claim 2 wherein said protein is chosen from at least one of sericin, bovine serum albumin, gelatin, egg-albumin, casein, zein, whey or lysozyme.

4. A composition as claimed in any one of the preceding claims wherein said crystals of zinc oxide are shaped like a disc.

5. A composition as claimed in any one of the preceding claims wherein said zinc oxide has a formula ZnOₓ where x<1 and **characterized by** a photoluminescence peak at 452 nm.

6. A composition as claimed in any one of the preceding claims comprising 0.01 to 10%, preferably 0.01 to 5%, more preferably 0.05 to 2% zinc pyrithione by weight of the composition.

7. A composition as claimed in any one of the preceding claims comprising 0.05 to 5%, of said habit modified crystals of zinc oxide.

8. A composition as claimed in any one of the preceding claims wherein the weight ratio of the habit modified crystals of zinc oxide to zinc pyrithione is less than or equal to 1:1.

9. A composition as claimed in any one of the preceding claims additionally comprising a cosmetically acceptable carrier.

10. A composition as claimed in any one of the preceding claims wherein said composition is a personal cleansing composition.

11. A composition as claimed in claim 10 wherein said composition is a shampoo, hair conditioner or a body wash composition.

12. A process to prepare a composition as claimed in claim 1 comprising the step of mixing zinc pyrithione with said habit modified crystals of zinc oxide; said habit modified crystals of zinc oxide are prepared by a process comprising the steps of:
(i) mixing aqueous solution of a water soluble protein with aqueous solution of a non-hydrolysable water-soluble zinc salt;
(ii) adding a weak base thereto;
(iii) heating the reaction mixture of step (ii) to attain temperature of 60 to 90 °C; and,
(iv) filtering the contents of step (iii), washing the residue with water, and drying it to obtain said habit modified crystals of zinc oxide as the filtrate.

13. A process as claimed in claim 12 wherein heated reaction mixture of step (iii) is autoclaved under pressure of 4 to 6 bar in an inert atmosphere.

14. A process as claimed in claim 12 or 13 wherein said habit modified crystals of zinc oxide are further calcined at 600 °C to 800 °C.

15. Non-therapeutic use of habit modified crystals of zinc oxide; and zinc pyrithione;
wherein the ratio of photoluminescence peak intensity at 452 nm of the habit modified crystals of zinc oxide to that of zinc oxide is at least 5; and
wherein the habit modified crystals of zinc oxide are shaped like a disc; the thickness of each disc is from 10 nm to 30 nm and the average diameter of the disc is in the range of 200 nm to 500 nm for manufacture of a composition for providing antimicrobial benefit.

## Patentansprüche

1. Antimikrobielle Zusammensetzung, umfassend
(a) Habitus-modifizierte Kristalle von Zinkoxid; und
(b) Zinkpyrithion,
wobei das Verhältnis der Intensität des Photolumineszenzpeaks bei 452 nm der Habitus-modifizierten Kristalle von Zinkoxid zu der des Zinkoxids mindestens 5 beträgt; und
wobei die Habitus-modifizierten Kristalle von Zinkoxid die Form einer Scheibe haben; die Dicke jeder Scheibe 10 nm bis 30 nm und der durchschnittliche Durchmesser der Scheibe in dem Bereich von 200 nm bis 500 nm liegt.

2. Antimikrobielle Zusammensetzung, wie im Anspruch 1 beansprucht, umfassend
(a) Habitus-modifizierte Kristalle von Zinkoxid, umfassend eingeschlossene Partikel eines wasserlöslichen Proteins; und
(b) Zinkpyrithion.

3. Zusammensetzung, wie im Anspruch 2 beansprucht, wobei das Protein aus mindestens einem von Sericin, Rinderserumalbumin, Gelatine, Eialbumin, Casein, Zein, Molke oder Lysozym ausgewählt ist.

4. Zusammensetzung, wie in irgendeinem der vorhergehenden Ansprüche beansprucht, wobei die Kristalle von Zinkoxid die Form einer Scheibe haben.

5. Zusammensetzung, wie in irgendeinem der vorhergehenden Ansprüche beansprucht, wobei das Zinkoxid eine Formel ZnOₓ aufweist, in der x<1 ist und durch einen Photolumineszenzpeak bei 452 nm gekennzeichnet ist.

6. Zusammensetzung, wie in irgendeinem der vorhergehenden Ansprüche beansprucht, umfassend 0,01 bis 10%, vorzugsweise 0,01 bis 5%, bevorzugter 0,05 bis 2% Zinkpyrithion, bezogen auf das Gewicht der Zusammensetzung.

7. Zusammensetzung, wie in irgendeinem der vorhergehenden Ansprüche beansprucht, umfassend 0,05 bis 5% der Habitus-modifizierten Kristalle von Zinkoxid.

8. Zusammensetzung, wie in irgendeinem der vorhergehenden Ansprüche beansprucht, wobei das Gewichtsverhältnis der Habitus-modifizierten Kristalle von Zinkoxid zu Zinkpyrithion kleiner als oder gleich 1:1 ist.

9. Zusammensetzung, wie in irgendeinem der vorhergehenden Ansprüche beansprucht, zusätzlich umfassend einen kosmetisch akzeptablen Träger.

10. Zusammensetzung, wie in irgendeinem der vorhergehenden Ansprüche beansprucht, wobei die Zusammensetzung eine Körperreinigungszusammensetzung ist.

11. Zusammensetzung, wie im Anspruch 10 beansprucht, wobei die Zusammensetzung ein Shampoo, ein Haarkonditionierer oder eine Körperwaschzusammensetzung ist.

12. Verfahren zur Herstellung einer Zusammensetzung, wie im Anspruch 1 beansprucht, umfassend den Schritt des Mischens von Zinkpyrithion mit den Habitus-modifizierten Kristallen von Zinkoxid, wobei die Habitus-modifizierten Kristalle von Zinkoxid durch ein Verfahren hergestellt werden, umfassend die Schritte:
(i) Mischen einer wässrigen Lösung eines wasserlöslichen Proteins mit einer wässrigen Lösung eines nicht-hydrolysierbaren wasserlöslichen Zinksalzes;
(ii) Zugeben einer schwachen Base;
(iii) Erhitzen der Reaktionsmischung von Schritt (ii), um eine Temperatur von 60 bis 90°C zu erreichen; und
(iv) Filtern des Inhalts von Schritt (iii), Waschen des Rückstands mit Wasser und Trocknen, um die Habitus-modifizierten Kristalle von Zinkoxid als Filtrat zu erhalten.

13. Verfahren, wie im Anspruch 12 beansprucht, wobei die erhitzte Reaktionsmischung von Schritt (iii) unter einem Druck von 4 bis 6 bar in einer inerten Atmosphäre autoklaviert wird.

14. Verfahren, wie im Anspruch 12 oder 13 beansprucht, wobei die Habitus-modifizierten Kristalle von Zinkoxid weiter bei 600°C bis 800°C calciniert werden.

15. Nicht-therapeutische Verwendung von Habitus-modifizierten Kristallen von Zinkoxid und Zinkpyrithion,
wobei das Verhältnis der Intensität des Photolumineszenzpeaks bei 452 nm der Habitus-modifizierten Kristalle von Zinkoxid zu der des Zinkoxids mindestens 5 beträgt; und
wobei die Habitus-modifizierten Kristalle von Zinkoxid die Form einer Scheibe haben; die Dicke jeder Scheibe 10 nm bis 30 nm und der durchschnittliche Durchmesser der Scheibe in dem Bereich von 200 nm bis 500 nm liegt, zur Herstellung einer Zusammensetzung zum Bereitstellen eines antimikrobiellen Nutzens.

## Revendications

1. Composition antimicrobienne comprenant
(a) des cristaux d'oxyde de zinc à habitus modifié ; et
(b) de la pyrithione de zinc
dans laquelle le rapport d'intensité de pic de photoluminescence à 452 nm des cristaux d'oxyde de zinc à habitus modifié à celle d'oxyde de zinc est d'au moins 5 ; et
dans laquelle les cristaux d'oxyde de zinc à habitus modifié sont formés comme un disque ; l'épaisseur de chaque disque est de 10 nm à 30 nm et le diamètre moyen du disque se trouve dans l'intervalle de 200 nm à 500 nm.

2. Composition antimicrobienne selon la revendication 1 comprenant
(a) des cristaux d'oxyde de zinc à habitus modifié comprenant des particules piégées d'une protéine soluble dans l'eau ; et
(b) de la pyrithione de zinc.

3. Composition selon la revendication 2, dans laquelle ladite protéine est choisie parmi au moins une de séricine, albumine de sérum bovin, gélatine, albumine d'oeuf, caséine, zéine, petit-lait ou lysozyme.

4. Composition selon l'une quelconque des revendications précédentes, dans laquelle lesdits cristaux d'oxyde de zinc sont façonnés comme un disque.

5. Composition selon l'une quelconque des revendications précédentes, dans laquelle ledit oxyde de zinc présente une formule ZnOₓ où x<1 et **caractérisée par** un pic de photoluminescence à 452 nm.

6. Composition selon l'une quelconque des revendications précédentes comprenant de 0,01 à 10 %, de préférence de 0,01 à 5 %, encore mieux de 0,05 à 2 % de pyrithione de zinc en masse de la composition.

7. Composition selon l'une quelconque des revendications précédentes comprenant de 0,05 à 5 %, desdits cristaux d'oxyde de zinc à habitus modifié.

8. Composition selon l'une quelconque des revendications précédentes, dans laquelle le rapport en masse des cristaux d'oxyde de zinc à habitus modifié à pyrithione de zinc est inférieur ou égal à 1:1.

9. Composition selon l'une quelconque des revendications précédentes comprenant de plus un support cosmétiquement acceptable.

10. Composition selon l'une quelconque des revendications précédentes, dans laquelle ladite composition est une composition pour l'hygiène personnelle.

11. Composition selon la revendication 10, dans laquelle ladite composition est un shampoing, un après-shampoing ou une composition pour le lavage du corps.

12. Procédé pour la préparation d'une composition selon la revendication 1 comprenant l'étape de mélange de pyrithione de zinc avec lesdits cristaux d'oxyde de zinc à habitus modifié ; lesdits cristaux d'oxyde de zinc à habitus modifié sont préparés par un procédé comprenant les étapes de :
(i) mélange de solution aqueuse d'une protéine soluble dans l'eau avec une solution aqueuse d'un sel de zinc soluble dans l'eau non-hydrolysable ;
(ii) addition d'une base faible à celle-ci ;
(iii) chauffage du mélange réactionnel de l'étape (ii) pour atteindre une température de 60 à 90°C ; et,
(iv) filtration des contenus de l'étape (iii), lavage du résidu avec de l'eau, et séchage de celui-ci pour obtenir lesdits cristaux d'oxyde de zinc à habitus modifié comme le filtrat.

13. Procédé selon la revendication 12, dans lequel le mélange réactionnel chauffé de l'étape (iii) est autoclavé sous une pression de 4 à 6 bar dans une atmosphère inerte.

14. Procédé selon la revendication 12 ou 13, dans lequel lesdits cristaux d'oxyde de zinc à habitus modifié sont de plus calcinés à de 600°C à 800°C.

15. Utilisation non-thérapeutique de cristaux d'oxyde de zinc à habitus modifié ; et de pyrithione de zinc ;
dans laquelle le rapport d'intensité de pic de photoluminescence à 452 nm des cristaux d'oxyde de zinc à habitus modifié à celle d'oxyde de zinc est d'au moins 5 ; et
dans laquelle les cristaux d'oxyde de zinc à habitus modifié sont façonnés comme un disque ; l'épaisseur de chaque disque est de 10 nm à 30 nm et le diamètre moyen du disque se trouve dans l'intervalle de 200 nm à 500 nm pour une fabrication d'une composition destinée à fournir un bénéfice antimicrobien.
